(19) Europäisches Patentamt
European Patent Office
Office européen des brevets

(11) Veröffentlichungsnummer: **0 261 327**
**A1**

(12) **EUROPÄISCHE PATENTANMELDUNG**

(21) Anmeldenummer: **87109573.3**

(22) Anmeldetag: **03.07.87**

(51) Int. Cl.4: **C07C 39/10**, C07C 37/60, C07C 43/23, C07C 41/26

(30) Priorität: **20.09.86 DE 3632075**

(43) Veröffentlichungstag der Anmeldung:
**30.03.88 Patentblatt 88/13**

(84) Benannte Vertragsstaaten:
**BE DE FR GB NL**

(71) Anmelder: **Degussa Aktiengesellschaft**
**Weissfrauenstrasse 9**
**D-6000 Frankfurt am Main 1(DE)**

(72) Erfinder: **Prescher, Günter, Dr.**
**Liesingstrasse 2**
**D-6450 Hanau 9(DE)**
Erfinder: **Ritter, Gebhard, Dr.**
**Fürstenbergstrasse 2**
**D-6450 Hanau 9(DE)**
Erfinder: **Sauerstein, Holger**
**Goethestrasse 1**
**D-6451 Grosskrotzenburg(DE)**

(54) **Verfahren zur Herstellung von Trihydroxy- bzw. Alkoxyhydroxybenzolen.**

(57) Resorcin und substituierte Resorcine lassen sich zu 1,2,3-und 1,2,4-Trihydroxybenzolen mit benzolischer Perpropionsäure in einer einzigen Reaktionsstufe umsetzen. Die Ausbeuten sind gut.

EP 0 261 327 A1

# Verfahren zur Herstellung von Trihydroxy-bzw. Alkoxy-hydroxybenzolen

Die Erfindung betrifft die Herstellung von Trihydroxy-und Alkoxy-hydroxybenzolen durch Hydroxylierung der um eine Hydroxylgruppe ärmeren Ausgangsverbindungen mittels benzolischer Perpropionsäure.

Trihydroxy-und Alkoxy-hydroxybenzole sind wichtige Synthesebausteine für Produkte in der Pharmazie und Kosmetik, sowie für Agro-und Fotochemikalien (Kirk. Othmer, Encyclopadia Chem.Technol., 3. Auflage, Band 18, Seite 670-84, 1982).

Bisher konnten diese Verbindungen nur sehr aufwendig über mehrere Verfahrensstufen hergestellt werden.

Nach der EP-PS 25 659 wurden Pyrogallol und substituierte Pyrogallolverbindungen zunächst durch Chlorierung von 2,6-Dimethylphenolabkömmlingen in entsprechende Chlorverbindungen überführt, deren Chlormethylengruppen dann in Aldehyde umgewandelt wurden. Die Aldehyde wurden dann entsprechend der Dakin-Reaktion mit Perverbindungen, wie Wasserstoffperoxid oder Persäuren, bzw. deren Salzen, in basischem Medium oxydiert zu den entsprechenden Pyrogallol-und substituierten Pyrogallolverbindungen.

Dieses mehrstufige Verfahren führte trotz des Aufwandes nur zu mäßigen Ausbeuten.

Ein weiteres mehrstufiges Verfahren über eine modifizierte Dakin-Reaktion, die über Hydroxybenzaldehyde verläuft, führte nur dann zu zufriedenstellenden Ausbeuten, wenn der pH-Wert während der Reaktion kontinuierlich auf 6 - 7 durch Basenzugabe gehalten wurde. Hierdurch wurde auch die Aufarbeitung erschwert (EP-PS 44 260).

Ein ebenfalls umständliches Verfahren mit sehr schwankenden Ausbeuten an Pyrogallol oder Pyrogallolverbindungen wird in der DE-OS 26 53 446 beschrieben. Ein 2,2,6,6-Tetrahalogencyclohexanon muß zunächst hergestellt werden, das dann mit Wasser in Gegenwart eines Katalysators oder in Form eines Alkoxyds mit Säure hydrolysiert wird.

Auch Versuche, über Diaminophenole zu Polyhydroxyverbindungen zu gelangen, führten nicht zu guten Ergebnissen und waren recht aufwendig (DE-OS 24 43 336).

Eine weitere Möglichkeit, über die Thiele-Winter-Reaktion zu Hydroxyhydrochinonen bzw. substituierten Polyhydroxyverbindungen zu kommen, war gleichzeitig sehr umständlich und technisch aufwendig. Die Reaktion besteht ja in der Umwandlung eines entsprechenden Chinons in ein Hydroxyhydrochinon durch dessen Acetoxylierung mit Essigsäureanhydrid in Gegenwart von Schwefelsäure oder Borfluoridätherat zu dem entsprechenden Triacetat und dann dessen Umwandlung in das gewünschte Hydroxyhydrochinon bzw. in substituierte Polyhydroxyverbindungen (DE-OS 24 59 059).

Bisher erfolgte die Darstellung von 2,6-Dimethoxyphenol durch vollständige Methylierung von der Gallussäure, anschließender Decarboxylierung und selektiver Entmethylierung der 2-Methoxyfunktion. Die Ausbeute an 2,6-Dimethoxyphenol war niedrig, da im ersten Schritt der Decarboxylierung nur eine Ausbeute von 50 % erreicht werden konnte, (J.Am.Chem.Soc. 72, 1950 , Seite 4986). Ein weiteres Verfahren wird in der Literaturstelle J.Org.Chem. 1984, 49, 4740 und J.Chem.Soc. Perkin Trans I 1974, Seite 1353, beschrieben.

Bei diesem Verfahren werden aromatische Aldehydverbindungen durch eine Baeyer-Villiger-Oxidation in die entsprechenden Phenole mit Wasserstoffperoxid oder Persäuren umgewandelt. Obwohl hierbei zum Teil gute Ausbeuten erhalten werden, hat dieses Verfahren den Nachteil der sehr langen Reaktionsdauer, d.h. bis zu 14 bzw. 16 Stunden. Außerdem erfordert auch dieses Verfahren mehrere Synthesestufen.

Aufgabe der Erfindung ist es daher, ausgehend von Resorcin und substituierten Resorcinen, direkt zu den entsprechenden Trihydroxyverbindungen bzw. Alkoxyhydroxybenzolen zu kommen.

Es wurde nun gefunden, daß sich diese Aufgabe lösen läßt, wenn man Resorcine der Formel

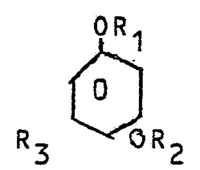 ,

in der $R_1$ und $R_2$ Wasserstoff, aliphatische gesättigte oder verzweigte Kohlenwasserstoffreste mit 1 - 9 Kohlenstoffatomen, welche gleich oder verschieden sein können, $R_3$ Wasserstoff oder die Gruppe - COOH, - $COOR_4$, $-CH_2-OR_4$, - $SO_3H$, - $NO_2$, - $NH_2$, - F, - Cl, - Br, - J oder $R_1$ bedeuten kann, wobei $R_4$ die gleiche Bedeutung wie $R_1$ besitzt, mit einer Lösung von Perpropionsäure in Benzol im Molverhältnis von 2 : 1 bis 20 : 1 (Resorcin der obengenannten Formel zu Perpropionsäure) bei einer Temperatur von 20 bis 140 °C umsetzt.

Benzolische Perpropionsäurelösungen werden z.B. nach dem Verfahren der DE-PS 25 19 289 erhalten. Diese Lösungen können zwar nach DE-PS 25 19 290 noch weiter gereinigt und in dieser Form eingesetzt werden; bevorzugt sind aber benzolische Rohextrakte aus der Herstellung der

Perpropionsäure, die noch Propionsäure, Wasserstoffperoxid und Mineralsäure, bevorzugt Schwefelsäure, enthalten. Die Konzentration der benzolischen Lösungen an Perpropionsäure liegen zwischen 2 und 60 Gew.-%, bevorzugt zwischen 10 bis 30 Gew.-%. Wird der Rohextrakt eingesetzt, so kann er bis zu 1,5 Gew.-% Wasserstoffperoxid; 1,5 Gew.-% Wasser und bis zu 800 ppm Mineralsäure enthalten. Der Gehalt an Propionsäure dieses Rohextrakts beträgt im allgemeinen bis zu 13 Gew.-%, obwohl auch etwas höhere Propionsäuregehalte weder bei der Durchführung der Reaktion, noch bei der späteren Aufarbeitung des Reaktionsgemisches schaden.

Neben Resorcin selbst eignen sich für das erfindungsgemäße Verfahren z.B. vor allem 2-Methylresorcin, 5-Methylresorcin (Orcin), 4n-Hexylresorcin, 4-t-Butylresorcin, 4-Chlorresorcin, Dihydroxybenzoesäure, Resorcindimethylether, Resorcinmonomethylether und Resorcindiäthylether. Besonders bevorzugt sind Resorcin, Resorcindimethylether, sowie auch Dihydroxybenzoesäure oder 2-Methylresorcin.

Die einzusetzenden Resorcine und substituierten Resorcinverbindungen liegen im allgemeinen in käuflicher Reinheit vor. Sie werden - falls sie fest sind - als Schmelze eingesetzt, solange ihr Schmelzpunkt so niedrig liegt, daß sie mit benzolischer Perpropionsäure keine sicherheitsbedenkliche Mischungen bilden. Die hochschmelzenden Resorcine dagegen werden mit organischen Lösungsmitteln aufgelöst und in flüssiger Phase zur Reaktion gebracht. Als Lösungsmittel kommen übliche Lösungsmittel, wie aliphatische Carbonsäureester, z.B. Essigsäuremethyl-, n-äthyl-, n-propyl-, n-butyl-isopropylester in Frage, außerdem Acetonitril und chlorierte Kohlenwasserstoffe.

Ein bevorzugtes Molverhältnis des betreffenden Resorcins zu Perpropionsäure liegt bei 2,5 bis 10 : 1. Bevorzugte Reaktionstemperaturen sind 30 - 120 °C.

Nicht entscheidend für den Verlauf der Reaktion ist der Druck; bevorzugt wird bei Atmosphärendruck gearbeitet, obwohl Unter-oder Überdruck anwendbar ist.

Der technische Fortschritt des erfindungsgemäßen Verfahrens liegt in der Möglichkeit, ausgehend von handelsüblichen Ausgangsstoffen in einer einzigen Reaktionsstufe in guten Ausbeuten,zu den gewünschten 1, 2, 3 und 1, 2, 4-Trihydroxybenzolen, beziehungsweise substituierten Hydroxy-Rersorcin-Verbindungen, zu kommen.
So kommt man z.B. in einem einzigen Reaktionsschritt zu Pyrogallol und Hydroxyhydrochinon, wie auch zu 2,6-Dimethoxyphenol und 2,4-Dimethoxyphenol. Die Umsätze der Perpropionsäure sind sehr hoch, häufig quantitativ. Ebenso sind die Ausbeuten wesentlich höher über denen der bekannten Verfahren.

Die Erfindung wird an den folgenden Beispielen erläutert:

<u>Beispiel 1</u> (Molverhältnis Resorcin zu Perpropionsäure = 20 : 1)

55 g (0,5 mol) Resorcin werden auf 110 °C erwärmt. Zu dieser gerührten Schmelze gibt man innerhalb 15 Minuten 10 g (22 gew.-%ige) benzolische Perpropionsäure (0,025 mol) hinzu. Die Temperatur erhöht sich danach auf 120 °C Nach Abklingen der Exotherme wird nach 30 Minuten ein Perpropionsäureumsatz von 100 % bestimmt. Das Reaktionsgemisch enthält dann 1,6 g Hydroxyhydrochinon und 1,27 g Pyrogallol, was einer Gesamtausbeute von 91 %, bezogen auf die eingesetzte Perpropionsäure, entspricht.

<u>Beispiel 2</u> (Molverhältnis Resorcin zu Perpropionsäure = 10 : 1)

55 g (0,5 mol) Resorcin werden auf 11o °C erwärmt. Zu dieser gerührten Schmelze gibt man innerhalb 15 Minuten 20,5 g (22 gew.-%ige) benzolische Perpropionsäure (0,051 mol) hinzu. Die Temperatur erhöht sich danach auf 120 °C. Nach Abklingen der Exotherme wird nach 30 Minuten ein Perpropionsäureumsatz von 100 % bestimmt. Das Reaktionsgemisch enthält dann 4,85 g Hydroxyhydrochinon und 1,16 g Pyrogallol, was einer Gesamtausbeute von 95 %, bezogen auf die eingesetzte Perpropionsäure, entspricht.

<u>Beipiel 3</u> (Molverhältnis Resorcin zu Perpropionsäure = 5 : 1)

55 g (0,5 mol) Resorcin werden auf 110 °C erwärmt. Zu dieser gerührten Schmelze gibt man innerhalb 25 Minuten 42 g (22 gew.-%ige) benzolische Perpropionsäure hinzu. Die Temperatur erhöht sich danach auf 120 °C. Nach Abklingen der Exotherme wird nach 45 Minuten ein Perpropionsäureumsatz von 100 % bestimmt. Das Reaktionsgemisch enthält dann 9,2 g Hydroxyhydrochinon und 2,5 g Pyrogallol, was einer Gesamtausbeute von 92 %, bezogen auf die eingesetzte Perpropionsäure, entspricht.

Beispiel 4 (Molverhältnis Resorcin zu Perpropionsäure = 2,5 : 1)

27,5 g (0,25 mol) Resorcin werden auf 110 °C erwärmt. Zu dieser gerührten Schmelze gibt man innerhalb 25 Minuten 41,0 g (22 gew.-%ige) benzolische Perpropionsäure. Durch die Zugabe der Persäure steigt die Temperatur innerhalb 2 Minuten auf 115 °C an und fällt dann nach 4 Minuten auf 90 °C ab. Nach 60 Minuten wird ein Perpropionsäureumsatz von 100 % bestimmt. Das Reaktionsgemisch enthält dann 6,95 g Hydroxyhydrochinon und 2,4 g Pyrogallol, was einer Gesamtausbeute von 74 %, bezogen auf die eingesetzte Perpropionsäure, entspricht.

Beispiel 5 (Molverhältnis Resorcin zu Perpropionsäure = 10 : 1; Resorcin in methanolischer Lösung)

55 g (0,5 mol) Resorcin werden in 16 ml Methanol bei 80 °C gelöst. Zu dieser gerührten Lösung gibt man 20,4 g (22 gew.-%ige) benzolische Perpropionsäure (0,05 mol). Die Temperatur erhöht sich danach auf 90 °C. Nach Abklingen der Exotherme wird nach 60 Minuten ein Perpropionsäureumsatz von 100 % bestimmt. Das Reaktionsgemisch enthält dann 4,8 g Hydroxyhydrochinon und1,0 g Pyrogallol, was einer Gesamtausbeute von 92 %, bezogen auf die eingesetzte Perpropionsäure, entspricht.

Beispiel 6 (Molverhältnis Resorcindimethylether zu Perpropionsäure = 10 : 1)

69 g (0,50 mol) Resorcindimethylether werden auf 30 °C erwärmt. Zu dieser gerührten Flüssigkeit gibt man innerhalb 25 Minuten 20,4 g (22 gew.-%ige) (0,05 mol) benzolische Perpropionsäure. Nach 45 Minuten Reaktionsdauer bestimmt man einen Perpropionsäureumsatz von 100 %. Das Reaktionsgemisch enthält dann 4,0 g 2,4-Dimethoxyphenol und 1,5 g 2,6-Dimethoxyphenol, was einer Gesamtausbeute von 70,5 %, bezogen auf die eingesetzte Perpropionsäure, entspricht.

**Ansprüche**

1.) Verfahren zur Herstellung von substituierten 1,2,3-und 1,2,4-Trihydroxybenzolen, dadurch gekennzeichnet, daß man Resorcine der Formel

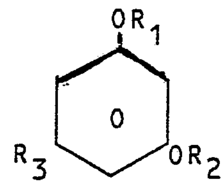

in der $R_1$ und $R_2$ Wasserstoff, aliphatische gesättigte oder verzweigte Kohlenwasserstoffreste mit 1 - 9 Kohlenstoffatomen, welche gleich oder verschieden sein können, $R_3$ Wasserstoff oder die Gruppe - COOH, - $COOR_4$ -$CH_2$ -$OR_4$, - $SO_3H$, - $NO_2$, - $NH_2$, - F, - Cl, - Br, - J oder $R_1$ bedeuten kann, wobei $R_4$ die gleiche Bedeutung wie $R_1$ besitzt, mit einer Lösung von Perpropionsäure in Benzol im Molverhältnis von 2 : 1 bis 20 : 1 (Resorcin der obengenannten Formel zu Perpropionsäure) bei einer Temperatur von 20 bis 140 °C umsetzt.

2.) Verfahren nach Anspruch 1, dadurch gekennzeichnet, daß man Resorcin selbst einsetzt, bevorzugt in Form seiner Schmelze.

3.) Verfahren nach Anspruch 1, dadurch gekennzeichnet, daß man als substituierte Resorcine 2-Methylresorcin, 5-Methylresorcin (Orcin), 4n-Hexylresorcin, 4-t-Butylresorcin, 4-Chlorresorcin, Dihydroxybenzoesäure, Resorcindimethylether, Resorcinmonomethylether und Resorcindiäthylether verwendet.

4.) Verfahren nach Anspruch 1 und 3, dadurch gekennzeichnet, daß man Resorcindimethylether, Dihydroxybenzoesäure oder 2-Methylresorcin verwendet.

5.) Verfahren nach Anspruch 1 bis 4, dadurch gekennzeichnet, daß man eine benzolische Perpropionsäurelösung mit einem Gehalt an Perpropionsäure von 2 - 60 Gew.-%, bevorzugt 10 - 30 Gew.-%, einsetzt.

6.) Verfahren nach Anspruch 1 bis 5, dadurch gekennzeichnet, daß man eine Perpropionsäurelösung mit einem Maximalgehalt von 1,5 Gew.-% Wasserstoffperoxid; 1,5 Gew.-% Wasser und bis zu 800 ppm Mineralsäure verwendet.

7.) Verfahren nach Anspruch 1 bis 6, dadurch gekennzeichnet, daß man das betreffende Resorcin und Perpropionsäure in einem Molverhältnis von 2,5 bis 10 einsetzt.

8.) Verfahren nach Anspruch 1 bis 7, dadurch gekennzeichnet, daß man die Reaktion bei 30 - 120 °C durchführt.

Europäisches Patentamt

# EUROPÄISCHER RECHERCHENBERICHT

Nummer der Anmeldung

EP 87109573.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - C3 - 2 658 943 (BAYER)<br>* Anspruch 1; Spalte 6, Zeilen 10-58 *<br>-- | 1,8 | C 07 C 39/10<br>C 07 C 37/60<br>C 07 C 43/23<br>C 07 C 41/26 |
| A | DE - C3 - 2 658 866 (BAYER)<br>* Anspruch 1; Spalte 9, Zeilen 7-55 *<br>-- | 1,8 | |
| A | DE - A1 - 3 031 736 (BAYER)<br>* Anspruch 1; Seite 9, Zeilen 24-27; Beispiel *<br>-- | 1 | |
| A | DE - A - 2 364 181 (AGENCY OF INDUSTRIAL SCIENCE & TECHNOLOGY et al.)<br>* Anspruch 1 *<br>-- | 1 | RECHERCHIERTE SACHGEBIETE (Int. Cl.4) |
| A | EP - A2 - 0 122 374 (DEGUSSA)<br>* Ansprüche 1-3 *<br>-- | 1 | C 07 C 39/00<br>C 07 C 37/00<br>C 07 C 43/00<br>C 07 C 41/00 |
| A | EP - A1 - 0 027 593 (BAYER)<br>* Ansprüche 1,8,9; Beispiel 3 *<br>-- | 1 | |
| A | DE - A - 2 138 735 (HAARMANN & REIMER)<br>* Anspruch 1 *<br>---- | 1 | |

Der vorliegende Recherchenbericht wurde für alle Patentansprüche erstellt.

| Recherchenort | Abschlußdatum der Recherche | Prüfer |
|---|---|---|
| WIEN | 09-12-1987 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82